# EUROPÄISCHE PATENTSCHRIFT

(11) **EP 1 744 776 B1**
(45) Veröffentlichungstag und Bekanntmachung des Hinweises auf die Patenterteilung: **29.08.2012**
(21) Anmeldenummer: 05716535.9
(22) Anmeldetag: 05.04.2005
(51) Int. Cl.: A61K 38/00

(54) **WUNDHEILUNGSFÖRDERNDE BOTENSTOFFMISCHUNG**
NEUROTRANSMITTER MIXTURE WHICH PROMOTES THE HEALING OF WOUNDS
MELANGE DE MESSAGERS FAVORISANT LA CICATRISATION

(30) Priorität: 06.04.2004 DE 102004018347
(43) Veröffentlichungstag der Anmeldung: 24.01.2007
(73) Patentinhaber: Schmolz, Dr., Manfred, 72810 Gomaringen (DE)
(72) Erfinder: SCHMOLZ, Manfred, 72810 Gomaringen (DE); KAUFMANN, Rolf, 63808 Haibach (DE); UHR, Günther, 04299 Leipzig (DE)
(74) Vertreter: Patentanwälte Ruff, Wilhelm, Beier, Dauster & Partner
(86) Internationale Anmeldenummer: PCT/EP2005/003551
(87) Internationale Veröffentlichungsnummer: WO 2005/097154

(56) Entgegenhaltungen:
- WO-A-01/38495
- WO-A-94/17904
- WO-A-99/24044
- WO-A-99/55346
- WO-A-02/056897
- DD-A1- 255 671
- US-A- 3 672 954
- US-A- 5 618 663
- READ M S ET AL: "PRESERVATION OF HEMOSTATIC AND STRUCUTRAL PROPERTIES OF REHYDRATED LYOPHILIZED PLATELETS: POTENTIAL FOR LONG-TERM STORAGE OF DRIED PLATELETS FOR TRANSFUSION" PROCEEDINGS OF THE NATIONAL ACADEMY OF SCIENCES OF USA, NATIONAL ACADEMY OF SCIENCE. WASHINGTON, US, Bd. 92, Nr. 2, Januar 1995 (1995-01), Seiten 397-401, XP001183381 ISSN: 0027-8424
- DOVGALEV S I ET AL: "PRODUCTION AND CLINICAL USE OF LYOPHILIZED THROMBOCYTES" ZDRAVOOKHRANENIE BELORUSSII, Bd. 17, Nr. 12, 1971, Seiten 39-41, XP009054686 ISSN: 0044-1961
- TOTSKAIA A A ET AL: "[A study of morphological peculiarities of lyophilized thrombocytes]" PROBLEMY GEMATOLOGII I PERELIVANIIA KROVI. MAR 1966, Bd. 11, Nr. 3, März 1966 (1966-03), Seiten 49-51, XP009054685 ISSN: 0552-2080
- FISCHER T H ET AL: "THROMBUS FORMATION WITH REHYDRATED, LYOPHILIZED PLATELETS" HEMATOLOGY, HARWOOD ACADEMIC PUBLISHERS, LUXEMBURG, LX, Bd. 7, Nr. 6, Dezember 2002 (2002-12), Seiten 359-369, XP009035962 ISSN: 1024-5332
- ANONYMOUS: "Arterial Blood Sampling Kits" INTERNET ARTICLE, [Online] 2004, XP002347630 Gefunden im Internet: URL:www.portexusa.com/abs/> [gefunden am 2005-10-04] -& ANONYMOUS: "Arterial Blood Sampling - Micropulse" INTERNET ARTICLE, [Online] XP002348493 Gefunden im Internet: URL:www.samples.portex.com/pages/prod_disp lay.asp?n=93,98> [gefunden am 2005-10-04]

## Beschreibung

Die Erfindung betrifft eine wundheilungsfördernde Botenstoffmischung. Durch diese wundheilungsfördernde Botenstoffmischung kann spezifisch die Wundheilung gefördert werden. Weiterhin betrifft die Erfindung ein Verfahren zur Gewinnung der wundheilungsfördernden Botenstoffmischung, die Verwendung dieser Botenstoffmischung sowie einen Kit zur Gewinnung dieser wundheilungsfördernden Botenstoffmischung.

Unter einer Wunde wird die Unterbrechung des Zusammenhangs von Körpergeweben mit oder ohne Substanzverlust, die durch mechanische Verletzung oder physikalisch bedingte Zellschädigung verursacht wird, verstanden. Dabei können verschiedene Formen von Wunden auftreten, so z. B.:
Mechanische Wunde: Diese werden durch äußere Gewalteinwirkung, vor allem als Schnitt- und Stichwunden (scharf schneidend bzw. spitz), Quetsch-, Platz-, Riß- und Schürfwunden (stumpf), Kratz- und Bißwunden (kombiniert scharf-stumpf) und als Schußwunden verursacht.
Thermische Wunden: Diese werden durch Einwirkung von Hitze (Verbrennung) oder Kälte (Erfrierung) verursacht.
Chemische Wunden: Diese werden vor allem durch Verätzung mit Säuren oder Laugen verursacht.
Strahlenbedingte Wunden: Diese entstehen durch Einwirkung aktinischer (Ultraviolettstrahlung) und ionisierender Strahlung.

Zur Regeneration von zerstörte Gewebe bzw. zum Verschluß einer Wunde ist dabei insbesondere die Neubildung von Bindegeweben und Kapillaren notwendig. Die physiologischen Vorgänge, die zur Regeneration des zerstörten Gewebes führen, werden als Wundheilung bezeichnet. Bei der primären Wundheilung erfolgt ein rascher und komplikationsloser Verschluß und weitgehende Regeneration des zerstörten Gewebes in Folge minimaler Bindegewebneubildung zwischen den gut durchbluteten und ggf. adaptierten Wundrändern einer sauberen Wunde. Bei Wunden mit weiter auseinanderliegenden (gequetschten oder nekrotischen Wundrändern) bzw. bei Wundinfektionen erfolgt eine verzögerte sekundäre Wundheilung. Bei dieser kann es in Folge einer bakteriellen Entzündung zur Auffüllung des Gewebedefekts mit Granulationsgewebe und einhergehend damit einem ausgedehnten Narbengewebe kommen. Die Epithelisierung vom Rand her bildet den Abschluß der Wundheilung.

Die Wundheilung selbst wird in verschiedenen Phasen unterteilt. In der ersten Phase, der sogenannten Latenzphase, kommt es zu a) einer exsudativen Phase mit Schorfbildung (in den ersten Stunden), und b) einer resorptiven Phase mit kataboler Autolyse (erster bis dritter Tag). In der zweiten Phase, der sogenannten Proliferationsphase, erfolgt eine anabole Reparation mit Bildung von Kollagen durch Fibroplasten (vierter bis siebter Tag). In der dritten Phase, der sogenannten Reparationsphase, erfolgt die Umwandlung des Granulationsgewebes in eine Narbe (ab dem achten Tag).

Bei der Wundheilung kann es in Folge einer bakteriellen Infektion einer Wunde, mit den klassischen Zeichen einer lokalen Entzündung, zu einer Wundinfektion kommen. Diese stellt eine unerwünschte Erscheinung bei der Wundheilung dar, da zu ihrer Therapie häufig eine Wundrevision, ein Abstrich zur mikrobiologischen Untersuchung, oder eine Wundreinigung mit Entfernung von Nekrosen, Fremdkörpern usw. nötig ist. Ferner muß häufig eine Spülung und Wunddrainage (Abfluß von Eiter und Wundsekret), sowie ein täglicher Wechsel antiseptischer Verbände, sowie ggf. die Zugabe von Antibiotika in Betracht gezogen werden.

Bei der Implantation, d. h. beim Einbringen oder Einpflanzen von körperfremden Materialien in den Organismus, kommt es häufig zu sogenannten Implantationsschäden. Unter Implantate wird die zusammenfassende Bezeichnung für Stoffe und Teile verstanden, die zur Erfüllung bestimmter Ersatzfunktion für einen begrenzten Zeitraum oder auf Lebenszeit in den menschlichen und/oder tierischen Körper eingebracht werden. Im Gegensatz zum Transplantat bestehen Implantate aus toter Materie (Alloplastik). Als Ersatzfunktion kommen vor allem die Unterstützung, Steuerung bzw. der partielle oder komplette Ersatz von Organfunktion (z. B. Linsenimplantation, künstlicher Herzschrittmacher, künstliche Herzklappen, Gefäßimplantate aus Kunststoff bei der rekonstruktiven Gefäßchirurgie, Endoprothese) die Unterstützung von Heilungsprozessen (z. B. Ruhigstellung einer Fraktur mittels Osteosynthese), die Übertragung von Kräften (z. B. übungsstabile und exakte Reposition von Frakturen mittels Osteosynthese), die Regulation von Deformitäten (z. B. Implantation von Harrington-Stäben zur Aufrichtung einer Skoliose) oder die plastische Raumausfüllung (z. B. Mammaplastik mittels Kunststoffimplantaten) bzw. Defektdeckung (z. B. Verschluß von Kalottendefekten mittels einer aus Kunststoff oder Metall bestehenden Schädelplatte) in Betracht. Bei Langzeitimplantaten tritt das Problem der Materialverträglichkeit in den Vordergrund. Weiterhin sind Implantate insbesondere in der Zahnmedizin gebräuchlich. Dort bestehen sie meistens aus aluplastischen Materialien (insbesondere Metall und Materialien mineralogischer Herkunft), die zum Aufbau verlorengegangener Knochensubstanz, zum Ersatz einzelner Zähne (Einzelimplantate), zur Versorgung mit festsitzenden Brücken bei verkürzter oder unterbrochener Zahnreihe und zur Stabilisierung totaler Prothesen dienen.

Zur Vermeidung von Implantationsschäden und zur generellen Förderung der Wundheilung sind eine Reihe von Mitteln bzw. Substanzen auf dem Markt. Bekannt sind dabei insbesondere die sogenannten Wundsalben, sowie in der Implantologie die Beschichtung bzw. die Auftragung von Lösungen auf ein Implantat vor oder während dessen Implantation. Diese Lösung(en) bestehen zum Teil aus Blut bzw. Blutbestandteilen.

Beispielsweise beschreibt die WO 99/24044 A1 ein die Wundheilung förderndes Arzneimittel, welches Thrombozyten bzw. wasserunlösliche Thrombozytenbestandteile enthält. Aus diesen können nach Zugabe physiologischer Stimuli wundheilungsfördernde Botenstoffe freigesetzt werden.

Die WO 02/056897 A2 offenbart ein Arzneimittel, insbesondere zur Förderung der Knochengeweberegeneration, das Mikropartikel enthält, welche von Blutzellen, darunter auch Thrombozyten, freigesetzt werden.

Aus der US 5,618,663 ist ein Verfahren zur Herstellung von Thrombozytenfaktoren bekannt. Dabei werden die Thrombozyten durch eine Aktivierungslösung zur Freisetzung der Faktoren veranlasst, während sie selbst durch Filtration zurückgehalten werden.

Die WO 01/38495 A2 beschreibt ein Verfahren zur Aufbereitung eines Thrombozytenpräparats, wobei die Thrombozytenrezeptoren für Thrombopoietin blockiert werden. Durch eine derartige Vorbehandlung wird eine *in vivo*-Absenkung des Thrombopoietinspiegels nach der Transfusion des Präparates vermieden und die eigene Blutbildung verstärkt.

Die WO 94/17904 A1 offenbart eine Membran für medizinische Zwecke, die durch Behandlung mit einem Niederdruckplasma modifiziert wird. Diese Behandlung führt zu einer Reduzierung der Komplementaktivierung sowie der Thrombogenität und damit zu einer geringeren Beeinträchtigung von an der Membran vorbeifließendem Blut.

Aus der DE 69320342 T2 (entsprechend WO93/25215 und EP 0643582 B1) ist bekannt, ein Thrombozytenkonzentrat auf einem Filter mit Aktivatoren zu behandeln, um die Thrombozyten zu veranlassen, Plättchenfaktoren freizusetzen, wonach die diese Faktoren enthaltende Aktivatorlösung durch Filtration von den Thrombozyten abfiltert wird.

Diese Form der Förderung der Wundheilung hat aber eine Reihe von Nachteilen. So ist z. B. bei Aufbringung einer Lösung während einer Operation die Gefahr einer möglichen Kontamination gegeben, insbesondere wenn das Blut zur Gewinnung des Thrombozytenkonzentrats zuvor zentrifugiert werden muss. Weiterhin muss das (entnommene) Blut zwischen dem Operationssaal und der Zentrifuge hin und her transportiert werden, was zu einer Veränderung der Blutbestandteile führen kann. Es hat sich auch gezeigt, dass die bloße Auftragung von Blut(bestandteilen) auf ein Implantat nur eine geringe Förderung der Wundheilung zur Folge hat. Dies gilt auch für Lösungen, die Botenstoffe bzw. Plättchenfaktoren enthalten, die durch Behandlung von Thrombozyten mit Aktivatoren gewonnen wurden. Zu dem enthalten solche Lösungen noch Aktivatoren.

Die Erfindung stellt sich daher die Aufgabe, eine verbesserte Botenstoffmischung bereitzustellen, welche eine wundheilungsfördernde Wirkung aufweist. Dabei sollen (spezifische) wundheilungsfördernde Botenstoffe bereitgestellt werden, die eine beschleunigte Wundheilung induzieren können. Ferner sollen die beschriebenen Nachteile des Standes der Technik umgangen werden.

Diese Aufgabe wird gelöst durch ein Verfahren zur Gewinnung einer wundheilungsfördernden Botenstoffmischung, wie sie in Anspruch 1 beschrieben ist. Bevorzugte Ausführungsformen sind in den Ansprüchen 2 bis 6 dargestellt. Die Ansprüche 7 bis 13 betreffen die Verwendung eines Membranfilters zur Gewinnung der wundheilungsfördernden Botenstoffmischung. Die Ansprüche 14 bis 19 betreffen einen Kit zur Gewinnung der wundheilungsfördernden Botenstoffmischung. Der Wortlaut sämtlicher Ansprüche wird hiermit durch Bezugnahme zum Inhalt der Beschreibung gemacht.

Überraschenderweise wurde gefunden, dass durch Aufschluß von Thrombozyten der Thrombozyteninhalt als wundheilungsfördernde Botenstoffmischung gewonnen werden kann, deren wundheilungsfördernde Wirkung nach Entfernung unlöslicher Körperflüssigkeitsbestandteile verbessert ist.

Gegenstand der Erfindung ist somit eine wundheilungsfördernde Botenstoffmischung aus Thrombozyten insbesondere solche aus Blut oder Blutplasma. Diese ist vollständig frei, von in wässrigen Lösungsmitteln unlöslichen Bestandteilen, insbesondere unlöslichen Blutbestandteilen. Die wundheilungsfördernde Botenstoffmischung enthält im wesentlichen alle wasserlöslichen Botenstoffe aus Thrombozyten. Die erfindungsgemäße wundheilungsfördernde Botenstoffmischung kann auch im wesentlichen frei, vorzugsweise vollständig frei, sein von außerhalb von Thrombozyten in Körperflüssigkeiten vorhandenen wasserlöslichen Stoffen. Weiterhin kann die Botenstoffmischung in Form einer wässrigen Lösung vorliegen. Alternativ dazu kann die wundheilungsfördernde Botenstoffmischung in trockener, insbesondere lyophilisierter (gefriergetrockneter) und/oder pulvriger Form vorliegen.

In einer besonders bevorzugten Ausführungsform liegt die wundheilungsfördernde Botenstoffmischung, vorzugsweise die in wässriger Lösung vorliegende wundheilungsfördernde Botenstoffmischung, in steriler Form und ggf. steriler Verpackung vor. Bei den Botenstoffen kann es sich dabei sowohl um körpereigene (autologe) Botenstoffe, d. h. Botenstoffe, die von einem zu behandelnden Patienten selbst entnommenen wurden, als auch um körperfremde Botenstoffe handeln. Der Vorteil bei Nutzung körpereigener Botenstoffe besteht darin, dass eventuell auftretende Immunreaktionen oder Infektionen vermieden werden können. Sowohl bei Nutzung körperfremder Botenstoffe als auch bei Nutzung der körpereigenen Botenstoffe können zur Vermeidung einer Immunreaktion bzw. Infektion durch eventuell vorhandenen Antigene oder pathogenen Partikeln diese unschädlich gemacht werden. Dies kann während oder nach der Blutentnahme, aber vor der Gewinnung der Botenstoffe, erfolgen, aber auch eine Inaktivierung eventuell vorhandener pathogener Partikel nach Gewinnung der Botenstoffmischung ist vorzugsweise vorgesehen. Die erfindungsgemäße wundheilungsfördernde Botenstoffmischung kann auch auf Körperoberflächen zum Einsatz gebracht werden. Die erfindungsgemäße wundheilungsfördernde Botenstoffmischung ist frei von unlöslichen Blutbestandteilen, wie z.B. Zellwandmaterial(ien), die bekannte Antigene darstellen und eine Immunreaktion/Infektion induzieren können. Somit reicht es aus, falls nötig, bei Verwendung der erfindungsgemäßen wundheilungsfördernde Botenstoffmischung Immunsuppressiva, Antibiotika etc. in vergleichsweise geringen Konzentrationen hinzuzugeben, was die Verträglichkeit der Botenstoffmischung entscheidend verbessern hilft.

Allgemein kann es sich bei der wundheilungsfördernden Botenstoffmischung bzw. bei den wundheilungsfördernden Botenstoffen um solche handeln, welche die Gen-Expression und/oder die Funktion von Zellen, insbesondere solchen des Bindegewebes, des Immunsystems, der Haut und/oder der Knochen, beeinflussen und deren wundreparativen Aktivitäten stimulieren. Bei den Bindegewebszellen ist insbesondere an Fibroplasten und/oder Fibrozyten gedacht. Bei den Zellen des Immunsystems ist an solche gedacht, welche direkt oder indirekt mit den Zellen der Haut, der Schleimhäute und/oder des Bindegewebes kommunizieren und dadurch wundreparative Aktivitäten stimulieren. Vorzugsweise sind solche Zellen des Immunsystems z.B. Leukozyten, insbesondere Monozyten, Makrophagen, Granulozyten und/oder Lymphozyten. Bei den Hautzellen handelt es sich z.B. um Keratinozyten. Bei den Knochenzellen ist vorzugsweise an sogenannte Osteoplasten und/oder Osteoklasten gedacht. Die erfindungsgemäße Botenstoffmischung kann alle diese Zelltypen (insbesondere Bindegewebs-, Immun-, Haut- sowie Knochenzellen) zum einen einzeln, oder, je nach Wundtyp, Gewebetyp bzw. Einsatzort der Botenstoffmischung, auch zusammen beeinflussen, insbesondere stimulieren.

In einer besonders bevorzugten Ausführungsform handelt es sich bei den wundheilungsfördernden Botenstoffen um Mediatoren, insbesondere Mediatoren wie TGFβ (transforming growth factor β) PDGF (platelet derived growth factor), VEGF (vascular endothelial growth factor) und/oder IGF (insulin-like growth factor).

Gegenstand der Erfindung ist ein Verfahren zur Gewinnung der erfindungsgemäßen wundheilungsfördernden Botenstoffmischung, wobei in entnommenen Körperflüssigkeiten, insbesondere Blut oder Blutplasma, vorhandene Botenstoffe nach Trennung der Körperflüssigkeiten in Überstand und Sediment aus dem abgetrennten Überstand gewonnen werden. Die Gewinnung der Botenstoffe erfolgt durch Freisetzung des gesamten Inhalts der Thrombozyten durch deren Aufschluß. Dies ist ein wesentlicher Unterschied zur Freisetzung lediglich der in den Vesikeln der Thrombozyten enthaltenen Botenstoffe nach Zugabe von Aktivatoren, wie dies beim Verfahren nach der DE 693 20 342 T2 der Fall ist. Das Blut kann vor der Trennung in Überstand und Sediment mit mindestens einem Blutgerinnungsinhibitor in Verbindung gebracht werden. Als nutzbare Blutgerinnungsinhibitoren sind dem Fachmann eine ganze Reihe von Substanzen bekannt. So kann z. B. die Blutgerinnung physiologisch durch intaktes Gefäßendothel, Antithrombin III, Fibrinspaltprodukte, Heparin etc., pharmakologisch durch Antikoagulanzien und Fibrinolytika, gehemmt werden. Weitere Blutgerinnungsinhibitoren sind dem Fachmann bekannt und werden von der Erfindung ebenfalls umfasst.

In einer weiteren bevorzugten Ausführungsform wird die Trennung bzw. Sedimentation in Gegenwart mindestens eines Sedimentationsbeschleunigers, vorzugsweise eines Polysaccharids und/oder substituierten Polysaccharids, insbesondere Dextran, Dextransulfat und/oder Hydroxyethylstärke (HES) durchgeführt. Insbesondere bei Verwendung von Sedimentationsbeschleunigern kann die Sedimentation ohne (vorherige) Zentrifugation durchgeführt werden. Dabei kann insbesondere die Sedimentation ausschließlich durch die Einwirkung von natürlicher Schwerkraft erfolgen. So kann z.B. bei Nutzung der natürlichen Schwerkraft die Trennung in Überstand und Sediment durch einfaches Stehenlassen der zuvor entnommenen Blutprobe erfolgen. Bei dem Überstand kann es sich im wesentlichen um die Thrombozyten handeln, während das Sediment im wesentlichen die Erythrozytenfraktion des Blutes darstellt. Die Trennung der Thrombozyten- von der Erythrozytenfraktion kann z.B. im wesentlichen unmittelbar nach der Blutentnahme erfolgen. Die rasche Trennung von Thrombozyten- und Erythrozytenfraktion hat den Vorteil, dass inhibierend auf die Botenstoffmischung wirkende Faktoren aus der Probe rasch entfernt werden können. Dadurch haben die wundheilungsfördernden Botenstoffe die bestmögliche biologische Aktivität. Weiterhin kann die Aktivität der Botenstoffmischung, d. h. ihre stimulierende/fördernde Wirkung auf die Wundheilung, von der "Frische" der Blutprobe abhängig sein. Alternativ zu der unmittelbar auf die Blutentnahme folgenden Trennung der Thrombozyten- von der Erythrozytenfraktion kann auch eine Lagerung der Blutprobe, so z.B. durch Kühlung bzw. Konservierung, erfolgen. Wenn dann die gelagerte Probe bzw. deren Inhalt (d.h. die zuvor gewonnene wundheilungsfördernde Botenstoffmischung, die ebenfalls eingelagert sein kann) benötigt wird, kann die Gewinnung der wundheilungsfördernden Botenstoffmischung durchgeführt werden, oder aber eine schon gewonnene (und zwischenzeitlich eingelagerte) wundheilungsfördernde Botenstoffmischung selbst kommt zum Einsatz. Ein Verfahren zur "Konservierung" einer schon gewonnenen wundheilungsfördernde Botenstoffmischung stellt z.B. die Lyophilisierung (Gefriertrocknung) dar. Weitere Konservierungsmethoden sind dem Fachmann bekannt. Der Vorteil einer Lagerung bzw. Konservierung des Ausgangsmaterials und/oder der zuvor gewonnenen Botenstoffmischung besteht darin, dass bei einem absehbaren Bedarf, wie z.B. bei einer bevorstehenden Operation, im Vorfeld mit der Gewinnung der Botenstoffmischung begonnen werden kann. Mit dieser Vorgehensweise ist es möglich, größere Mengen an Ausgangsmaterial bzw. wundheilungsfördernde Botenstoffmischung(en) durch merhfache Entnahme kleiner Blutmengen zu gewinnen und diese für den Bedarfsfall zu lagern, ohne den Organismus zu sehr zu belasten (wie dies z.B. durch Entnahme größerer Mengen an Blut der Fall wäre), was für die Regeneration förderlich ist. Abhängig von der Aktivität der Botenstoffe kann entweder das Ausgangsmaterial oder die Botenstoffmischung selbst gelagert werden. Das als Überstand gewonnene thrombozytenhaltige Plasma wird durch Waschen mit einer Pufferlösung gereinigt. Dabei werden insbesondere die Thrombozyten gereinigt. Dem Fachmann sind eine Reihe von Pufferlösungen bekannt, die für solche eine Reinigung genutzt werden können.

Der Thrombozyteninhalt selbst kann dadurch freigesetzt werden, dass man die zuvor gewonnenen Thrombozyten mit einer wässrigen Flüssigkeit, Wasser oder Lösung, inkubiert, wobei die Inkubation mit einer derartigen Flüssigkeit zu einer Freisetzung der Mediatoren führt. Dabei wird der Thrombozyteninhalt durch den Aufschluss, insbesondere Lysierung, der Thormbozyten (z.B. durch Zugabe von destilliertem Wasser oder einer hypotonischen Lösung, Ansetzen eines Vakuums, Ultraschall, enzymatische Verdauung etc.) freigesetzt. Die wässrige Flüssigkeit besitzt vorzugsweise einen geringeren osmotischen Druck als der Thrombozyteninhalt. Eine diesbezügliche wässrige Flüssigkeit kann z. B. destilliertes Wasser sein. Die Thrombozyten können vor der Freisetzung der Mediatoren durch vorheriges Spülen mit einer physiologischen Pufferlösung im wesentlichen von den Resten der Lösung, bestehend aus Plasma, Sedimentationsbeschleuniger und Gerinnungsinhibitor, befreit werden.

In einer weiteren bevorzugten Ausführungsform wird der Inhalt der Thrombozyten in der Freisetzungsflüssigkeit aufgenommen, und die erhaltene Lösung, insbesondere wässrige Lösung, des Thrombozyteninhalts wird dann filtriert, insbesondere steril filtriert. Als Freisetzungsflüssigkeit kann insbesondere destilliertes Wasser dienen. Das Inkubieren der Thrombozyten in der Freisetzungslösung sowie die Freisetzung der Mediatoren und insbesondere auch das Waschen der Thrombozyten wird in einer weiteren bevorzugten Ausführungsform auf einem Filter, insbesondere einem Sterilfilter, vorgenommen. Das Waschen kann z. B. mit der (physiologischen) Pufferlösung, das Inkubieren der Thrombozyten mit destilliertem Wasser, erfolgen. Die Menge an Flüssigkeit bzw. Lösung, insbesondere an destilliertem Wasser ist vorzugsweise so bemessen, dass man die Thrombozyten, ggf. die Thrombozyten insbesondere auf dem (Steril-)Filter zum osmotischen Platzen bringt. Ferner kann die Menge an destilliertem Wasser bzw. generell die Menge einer Freisetzungslösung so bemessen sein, dass sich nach der Freisetzung des Thrombozyteninhalts, z. B. durch osmotisches Platzenlassen, eine isotonische Lösung des Thrombozyteninhalts in der Freisetzungslösung, z. B. in destilliertem Wasser, ergibt. Dies hat den Vorteil, dass die so gewonnene Botenstoffmischung direkt auf eine Wunde bzw. auf ein Implantat aufgetragen werden kann. Sofern keine isotonische Lösung vorliegt, können die freigesetzten Botenstoffe trotzdem direkt auf Implantate oder auf eine Wunde aufgetragen werden. Der eventuell geringe Unterschied des osmotischen Wertes zum physiologischen Wert im Gewebe kann gegebenenfalls ausgeglichen werden. Das osmotische Platzentassen der Thrombozyten hat den Vorteil, dass es mit geringem präparativen Aufwand durchgeführt werden kann. Die Durchführung des erfindungsgemäßen Verfahrens kann direkt im Operationssaal oder in einer Arztpraxis durchgeführt werden. So können die Blutentnahme, die Freisetzung des Thrombozyteninhaltes und/oder auch die Verwendung des Thrombozyteninhaltes, z. B. die Auftragung auf eine Wunde oder auf ein Implantat, direkt vor Ort durchgeführt werden. Dadurch kann Zeit gewonnen werden und eine mögliche Aktivitätsminderung der Botenstoffe oder eine Kontamination der Probe kann vermieden werden.

Weiterhin wird gemäß einer bevorzugten Ausführungsform die wundheilungsfördernde Botenstoffmischung aus Thrombozyten-enthaltenden Körperflüssigkeiten, insbesondere Blut oder Blutplasma, zur Beschichtung von Trägermaterialien, insbesondere Implantatmaterialien, verwendet. Als Implantatmaterialien können z. B. die in der Zahnheilkunde verwendeten Implantate, sowie Gefäßimplantate oder Organimplantate dienen. Bezüglich möglicher weiterer Implantate wird auf den bisherigen Text der Beschreibung Bezug genommen. Ferner wird mindestens ein Botenstoff aus Thrombozyten-enthaltenden Körperflüssigkeiten, insbesondere Blut oder Blutplasma, zur Beeinflussung, insbesondere Stimulierung, der Wundheilung im Bereich Haut, Schleimhaut, Gewebe und/oder Knochen gemäß einer besonders bevorzugten Ausführungsform verwendet. Dabei kann der Botenstoff bzw. die Botenstoffmischung sowohl alleine als auch in Kombination mit einem Implantat zur Anwendung kommen. Besonders bevorzugt ist die Verwendung eines Botenstoffes aus Thrombozyten-enthaltenden Körperflüssigkeiten, insbesondere Blut oder Blutplasma, zur Herstellung eines Medikamentes oder einer pharmazeutischen Zusammensetzung zur Beeinflussung, insbesondere Stimulierung, der Wundheilung. So können z. B. ein oder mehrere Botenstoffe zu einer Salbe verarbeitet werden, die dann auf Wunden aufgetragen wird.

In einer weiteren bevorzugten Ausführungsform wird ein Filter, insbesondere ein Sterilfilter, als Behältnis für die Freisetzung von Mediatoren aus Thrombozyten und zur Gewinnung einer wundheilungsfördernden Botenstoffmischung in Form eines sterilen wässrigen Filtrates verwendet. Dabei kann als Filter ein handelsüblicher Filter verwendet werden. Auch können, je nach Volumina der aufzuarbeitenden Thrombozyten-suspension, verschiedene Filterdurchmesser verwendet werden. So haben z. B. Filter für geringe Volumina an Plättchen-reichen Plasma ein kleines Totvolumen, so dass bei der Gewinnung der Mediatorenlösung die zur Ausschwemmung notwendigen Mengen an Spüllösung ebenfalls gering gehalten werden können. Für große Volumina an Plasma sind entsprechend große Filter, d.h. Filter mit größerer Filterfläche, geeignet. Passende Filter werden z. B. von der Firma Millipore Corp. vertrieben. Bei diesen Filtern besitzen verschiedene Größen jeweils das gleiche Material bei Filter bzw. Membran (Polyethersulfon) einerseits und Gehäuse (Acryl) anderseits. Ein erfindungsgemäßer Filter, insbesondere Sterilfilter, besitzt vorzugsweise eine geringe Probenbindung, d. h. Proteinbindung, um den Verlust an Material so gering wie möglich zu halten. An der Innenseite des Filters (Gehäuse und Membran) kann daher z.B. eine Beschichtung sein, die eine Gerinnung sowie eine Adhäsion von Thrombozyten verhindert oder zumindest verringert. Vorzugsweise ist dabei an eine kovalente Kopplung von Blutgerinnungsinhibitoren, z. B. Heparin, oder ähnlichen Materialien gedacht, wie sie z. B. bei zentralen Venenkathedern oder auch Stents eingesetzt werden. Ohne eine solche Beschichtung kann einiges an Mediatoren verloren gehen, weil die Thrombozyten beim Kontakt mit dem Filtermaterial aktiviert werden können. Die hierdurch bereits freigesetzten Mediatoren werden dann während des Spülvorgangs weggewaschen. Der Filter, insbesondere Sterilfilter, hat gemäß einer weiteren bevorzugten Ausführungsform eine Porengröße von 0,1 bis 0,5 µm, vorzugsweise von ca. 0,2 µm, besonders bevorzugt von ca. 0,22 µm. Generell ist bei der Auswahl des geeigneten Filters ein Filter bevorzugt, der möglichst große Mengen an Thrombozyten zurückhält. Die Thrombozyten (Blutplättchen) selbst sind im Knochenmark gebildete kernlose, scheibenförmige, corpuskuläre Blutbestandteile mit einem Durchmesser von 2 bis 3,5 µm und einer Dicke von 0,5 bis 0,75 µm. Daher sollte, damit die Thrombozyten auf dem Filter zurückgehalten werden, die Porengröße des Filters nicht größer als 0,5 µm sein. Passende Filter stellen z.B. die schon weiter oben benannten Filter der Firma Millipore Corp. dar., so die Filter Millex® GP, die aus Polyethersulfon bestehen und in Durchmessern von 25 mm oder 50 mm vertrieben werden und Membranen aus hydrophilem Polyethersulfon besitzen. Solche Filter haben eine hohe Durchlässigkeit und ein geringes Totvolumen, sodass mit kleinen Volumina gearbeitet werden kann.

Weiterhin wird erfindungsgemäß ein Kit zur Gewinnung von wundheilungsfördernden Botenstoffmischungen aus Thrombozyten-enthaltenden Körperflüssigkeiten mit einem Filter, insbesondere Sterilfilter beansprucht, wobei der a) Filter, insbesondere Sterilfilter, eine Porengröße von 0,1 µm bis 0,5 µm, vorzugsweise von ca. 0,2 µm, besonders bevorzugt von ca. 0,22 µm, aufweist. Dieser Kit wird zur Verwendung oder Durchführung des Verfahrens zur Gewinnung von Botenstoffmischungen genutzt. Weiterhin kann der Kit ein b) Blutentnahmebesteck mit c) Blutentnahmesystem, insbesondere einem Blutentnahmeröhrchen, insbesondere einem mit einem Sedimentationsbeschleuniger, vorzugsweise einem Polysaccharid und/oder einem substituierten Polysaccharid, insbesondere Dextran, Dextransulfat und/oder Hydroxyethylstärke (HES) vorgefüllte Blutentnahmeröhrchen oder einem Spritzenzylinder zur Blutentnahme, d) Blutgerinnungsinhibitor enthalten. Zu den weiteren Merkmalen eines Sedimentationsbeschleunigers, Blutgerinnungsinhibitors bzw. der Substanz zur Stimulierung der Freisetzung und/oder Generierung von Botenstoffen aus Thrombozyten wird auf den entsprechenden bisherigen Text der Beschreibung Bezug genommen. Der Kit kann auch e) eine Leerspritze, insbesondere eine Leerspritze mit einer passenden Kanüle zur Aufnahme der Thrombozyten-reichen Fraktion aus dem Überstand, f) ein Auffanggefäß, insbesondere ein steriles Auffanggefäß, besonders bevorzugt eine sterile Leerspritze zur Aufnahme der wundheilungsfördernden Botenstoffe nach Filtrierung, insbesondere nach Sterilfiltrierung, g) sowie ggf. einen oder zwei Dreiwegegenhähnen enthalten. Auch kann der Kit h) eine weitere Spritze, insbesondere eine mit physiologischer Pufferlösung vorgefüllte Spritze, sowie i) eine weitere Spritze, insbesondere eine mit Freisetzungslösung vorgefüllte Spritze, dem Kit enthalten. Der Blutgerinnungsinhibitor und/oder die Substanz zur Stimulierung der Freisetzung und/oder Generierung von wundheilungsfördernden Botenstoffen liegt in gemäß einer weiteren bevorzugten Ausführungsform vorzugsweise in flüssiger Form in dem Blutentnahmesystem, insbesondere in dem Blutröhrchen oder Spritzenzylinder, vor. Alternativ dazu kann schließlich in einer weiteren bevorzugten Ausführungsform der Blutgerinnungsinhibitor in vorzugsweise trockener, insbesondere lyophylisierter Form in dem Blutentnahmesystem, insbesondere in dem Blutröhrchen oder Spritzenzylinder, enthalten sein.

Die bestehenden Merkmale und weitere Merkmale der Erfindung ergeben sich aus der nachfolgenden Beschreibung von bevorzugten Ausführungsformen in Verbindung mit den Unteransprüchen. Hierbei können die einzelnen Merkmale jeweils für sich oder zu mehreren in Kombination miteinander verwirklicht sein.

In den Abbildungen zeigen:
- Fig. 1:: Eine mögliche erfindungsgemäße Ausführungsform zur Gewinnung einer wundheilungsfördernden Botenstoffmischung.
- Fig. 2:: Eine weitere mögliche erfindungsgemäße Ausführungsform zur Gewinnung einer wundheilungsfördernden Botenstoffmischung.

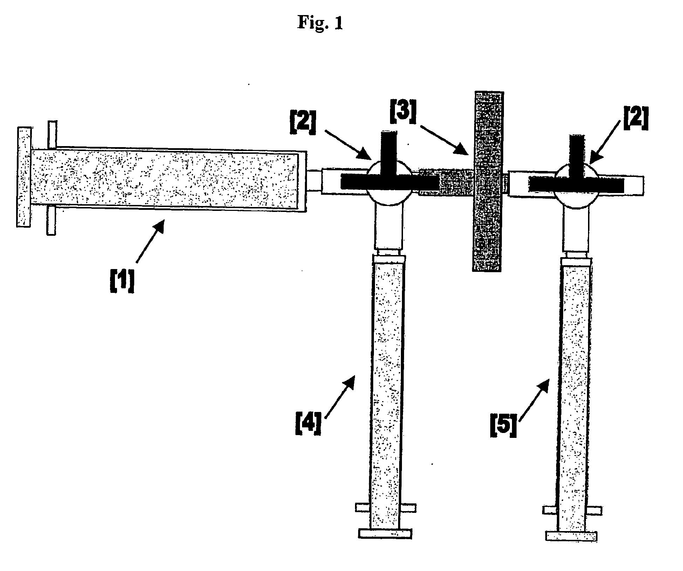

Fig. 1 zeigt eine schematische Darstellung einer möglichen Ausführungsform zur Gewinnung der erfindungsgemäßen wundheilungsfördernden Botenstoffmischung. Dabei sind drei Spritzen [1, 4, 5] an zwei 3-Wege-Hähnen [2] angeschlossen. Die zwei 3-Wege-Hähne [2] sind ihrerseits an einen Sterilfilter [3] angeflanscht. Die Flussrichtung für jede Spritze [1, 4, 5] kann separat eingestellt werden. Dabei werden die 3-Wege-Hähne derart umgelegt, dass die Flussrichtung für die einzustellende Spritze geschlossen oder in Richtung des Sterilfilters [3] geöffnet wird.

Die Spritze [1] (z.B. eine 20 ml Spritze), enthält das zuvor gewonnene Thrombozyten-reiche Plasma. Mittels des ersten 3-Wege-Hahnes [2] ist die Spritze [1] mit dem Sterilfilter [3] verbunden. Die zweite Spritze [4] ist ebenfalls mittels des ersten 3-Wege-Hahnes [2] mit dem Sterilfilter [3] verbunden. Die Flussrichtung in Richtung Sterilfilter [3] ist für die Spritze [4] geschlossen, wenn der 3-Wege-Hahn derart umgelegt wurde, dass die Flussrichtung für die Spritze [1] geöffnet wurde, und umgekehrt. Die Plättchensuspension, die in der Spritze [1] enthalten ist, wird auf den Sterilfilter aufgebracht und der flüssige Anteil der Plättchensuspension wird abfiltriert. Danach kann die Spritze [1] vom 3-Wege-Hahn [2] abgenommen und durch eine weitere Spritze ersetzt werden (nicht dargestellt), die eine Pufferlösung enthält, um so ggf. mittels dieser Pufferlösung den Sterilfilter [3] zu spülen.

Anschließend wird der 3-Wege-Hahn [2] derart umgelegt, dass die Flussrichtung von der Spritze mit der Plättchensuspension [1] bzw. Puffer-/Spüllösung geschlossen und die Flussrichtung von der Spritze mit der Lysislösung (z.B. destilliertes Wasser) [4] zum Sterilfilter [3] geöffnet wird. Nach Einwirkung der Lysislösung (z.B. destilliertes Wasser) werden die in den Plättchen enthaltenen Mediatoren freigesetzt, die mittels des zweiten 3-Wege-Hahnes [2], der den Sterilfilter [3] mit einem Auffanggefäß (z.B. Vakuum-Röhrchen mit Gummiseptum) [5] verbindet, in dem Auffanggefäß [5] eingesammelt werden. Diese stellen die gebrauchsfertige Mediatoren-Lösung dar. Sofern die in dem Auffanggefäß eingesammelte Mediatoren-Lösung modifiziert werden soll, kann die die Lysislösung enthaltende Spritze [4] abgezogen und durch eine weitere Spritze [nicht dargestellt] ersetzt werden, welche die gewünschten Modifikatoren (z.B. Vitamine, Spurenelemente, Pufferlösung etc.) enthält.

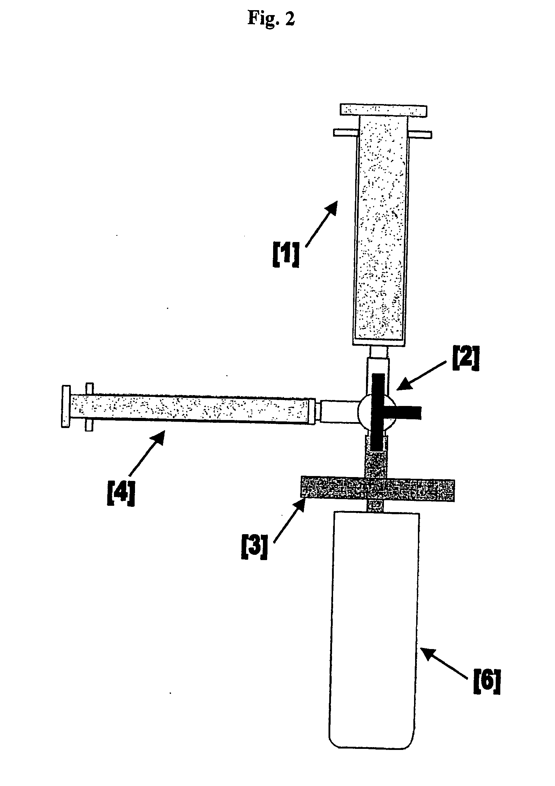

Fig. 2 zeigt eine weitere schematische Darstellung einer möglichen Ausführungsform zur Gewinnung der erfindungsgemäßen wundheilungsfördernden Botenstoffmischung. Dargestellt sind zwei Spritzen [1, 4], die an einen 3-Wege-Hahn [2] angeschlossen sind. Der Sterilfilter [3] ist an den 3-Wege-Hahn [2] angeflanscht. Das Auffanggefäß [6] ist direkt am Auslauf des Sterilfilters [3] angebracht. Die Flussrichtung für die beiden Spritzen [1, 4] kann ebenfalls, wie in Fig. 1 beschrieben, separat mittels des 3-Wege-Hahnes [2] eingestellt werden, und zwar derart, dass die Flussrichtung für die einzustellende Spritze geschlossen oder in Richtung des Sterilfilters [3] geöffnet wird, wobei jeweils immer nur eine der beiden Spritzen [1, 4] eine geöffnete Flussrichtung aufweist. Das Aufbringen der Plättchensuspension, die ggf. erwünschte Spülung der Suspension auf dem Sterilfilter sowie die Lysierung der Plättchen erfolgt im wesentlichen wie bei Fig. 1 beschrieben. Die Verbindung zwischen Sterilfilter [3] und Auffanggefäß [6] kann z.B. über einen Luer-Anschluß erfolgen, so dass die sterile Gewinnung der Mediatorenlösung im geschlossenen System möglich ist.

### Beispiel

### Durchführung:

Zunächst wird unter Beachtung von Vorsichtsmaßnahmen Blut entnommen (10 bis 50 ml). Insbesondere ist dabei an autologes Material gedacht, d. h. Material, das von derjenigen Person gewonnen wird, welche die Botenstoffmischung auch empfangen soll. Dadurch kann die Problematik bei Vorliegen nichterkannter Infektionen wie HIV, HBV, HCV, CMV etc. umgangen werden. Unter Wahrung der Sterilität zur Vermeidung einer Wundkontamination wird mit Hilfe eines Blutentnahmeröhrchens die Blutprobe entnommen. Bei diesem Blutentnahmeröhrchen handelt es sich um doppelfunktionelle Röhrchen (Monovetten oder Kabevetten), welche Spritze und Reagenzröhrchen in einem sind. Während der Entnahme vermischt sich das in das Röhrchen einströmende Blut mit dem bereits darin vorgegebenen Gemisch aus Gerinnungsinhibitor und HES im physiologischen Puffer. Die Vermischung wird noch dadurch verbessert, dass direkt nach Entnahme des Blutes das Röhrchen von der Entnahmekanüle angezogen, der Kolbenstempel der Spritze abgebrochen und das Röhrchen mehrfach sanft gekippt wird ("end-overend"). Direkt danach wird das Röhrchen aufrecht in einen entsprechenden Reagenzglasständer gestellt. Dort ruht es für 60 bis 120 Minuten. Im Anschluß daran wird das über dem Erythrozyten-Sediment stehende Thrombozyten-reiche Plasma mittels einer Leerspritze und einer extralangen Kanüle aufgesaugt, ohne dass rote Blutkörperchen aus dem Sediment mitgenommen werden. Dies wird durch Punktion des zuvor desinfizierten Gummiseptums im Deckel des Röhrchens, oder auch direkt auf dem Röhrchen (nach Entfernen des Deckels) gemacht. Anschließend wird die Kanüle von der Spritze abgezogen und die Thrombozyten-Suspension (mittels der Spritze) auf einen Sterilfilter aufgebracht. Hierzu wird die Spritze an einen 3-Wegehahn angeflanscht (gegenüber einer Spritze mit dem destillierten Wasser). Ein 25 mm-Filter kann dabei mit ca. 4 ml Thrombozyten-Suspension, ein 50 mm-Filter mit ca. 15 ml bis 20 ml Thrombozyten-Suspension, beschickt werden. Nach Abfiltirieren des flüssigen Anteils der Suspension wird die zum Aufbringen der Thrombozyten-Suspension verwendete Spritze vom 3-Wege-Hahn abgezogen und an deren Stelle die Spritze mit der Pufferlösung angebracht. Der Sterilfilter wird nun mittels dieser Pufferlösung gespült (Volumina von ca. 1 ml bis 20 ml). Hierzu wird die Pufferlösung ebenfalls durch den Filter gedrückt. Die Spritze kann im entleerten Zustand am 3-Wege-Hahn belassen werden. Schließlich wird das Auffanggefäß am Auslauf des Sterilfilters ausgetauscht (sofern das Anbringen des Auffanggefäßes über einen Luer-Anschluß erfolgt, ist die sterile Gewinnung der Mediatorenlösung im geschlossenen System möglich).

Alternativ können als Auffanggefäß Vakuum-Röhrchen mit Gummiseptum verwendet werden, so dass am Auslauf des Sterilfilters eine Kanüle aufgesetzt wird, welche sodann durch den Gummistopfen eines Vakuum-Röhrchens gestochen wird. Dabei wird Pufferlösung durch das Vakuum in das Auffangröhrchen gesaugt, was durch leichten Druck auf den Stempel der Spritze mit destilliertem Wasser unterstützt werden kann.

Danach wird der 3-Wege-Hahn so umgelegt, dass die Flußrichtung von der Spritze mit destilliertem Wasser zum Filter geöffnet wird. Sodann wird die Hälfte des destilliertem Wassers auf den Filter gedrückt. Dabei kann die Spritze einen Stopper enthalten, der nach Abgabe der Hälfte an destilliertem Wasser die restliche Flüssigkeitsmenge zurückhält. Nun wird der Filter (mit immer noch aufgesteckten Spritzen) für 5 Minuten in aufrechter Position inkubiert. Dies geschieht bei Raumtemperatur, die am Ort der Inkubation nicht über 40 °C und unter 4 °C liegen soll. Sodann wird der Rest des destillierten Wassers durch den Filter gedrückt. Durch die Einwirkung von destilliertem Wasser werden die Plättchen nicht wie üblich durch eine rezeptorabhängige Aktivierung zur Freisetzung der Mediatoren angeregt wie dies beim Verfahren nach der oben erwähnten DE 693 20 342 T2 der Fall ist, sondern durch osmotischen Druck zum Platzen gebracht, was zu einer vollständigeren und rascheren Freisetzung führt. Die so erhaltene Lösung im Auffanggefäß bildet die gebrauchsfertige Mediatoren-Lösung, mit welcher die Behandlung von Wunden oder die Beschichtung von Implantaten durchgeführt werden kann. Dadurch, daß keine Aktivatoren benötigt werden, die die Thrombozyten zum Freisetzung der Botenstoffe veranlassen, sondern die Freisetzung durch die Zerstörung der Thrombozyten erfolgt, enthält die Mediatorenlösung auch keine Aktivatoren. Die Menge an Freisetzungsflüssigkeit, insbesondere destilliertem Wasser ist vorzugsweise so berechnet worden, dass - zusammen mit den aus den aufgeschlossenen, insbesondere platzenden Thrombozyten freiwerdenden zellulären Inhalten - eine überwiegend physiologische Lösung (Proteingehalt, osmotischer Wert) gewonnen wurde. Aus 4 ml Thrombozyten-Suspension werden etwa 0,4 ml Botenstoff-Lösung und aus 20 ml Suspension ca. 2 ml Botenstofflösung erhalten. Die Ausbeute ist somit ca. 10% bezogen auf das volumen der Thrombozyten-Suspension.

Schließlich können durch Prüfung der Mediatoren-Lösung zu Qualitätskontrollzwecken Tests mittels einer geeigneten Methode durchgeführt werden. Als geeignete Testmethoden sind z. B. Konzentrationsbestimmungen der einzelnen Mediatoren im ELISA und/oder Messungen der biologischen Aktivität einzelner Mediatoren in zellulären Bioassays anzusehen.

## Patentansprüche

1. Verfahren zur Gewinnung einer wundheilungsfördernden Botenstoffmischung aus Thrombozyten enthaltenden Körperflüssigkeiten, insbesondere Blut oder Blutplasma, die frei ist von in wässrigem Lösungsmittel unlöslichen Bestandteilen, insbesondere unlöslichen Blutbestandteilen, wobei sie den wasserlöslichen Inhalt der Thrombozyten mit im Wesentlichen allen löslichen Botenstoffen der Thrombozyten enthält, wobei die in entnommenen Körperflüssigkeiten, insbesondere Blut oder Blutplasma, vorhandenen Botenstoffe nach Trennung der Körperflüssigkeiten in Überstand und Sediment aus dem abgetrennten Überstand gewonnen werden, indem man die Thrombozyten mit Pufferlösung wäscht, die Botenstoffe aus den Thrombozyten freisetzt und die erhaltene Lösung filtriert, wobei der Thrombozyteninhalt mit im Wesentlichen allen Botenstoffen der Thrombozyten durch Aufschluss der Thrombozyten freigesetzt wird, **dadurch gekennzeichnet, dass** der Aufschluss durch Lysierung der Thrombozyten vorgenommen wird.

2. Verfahren nach Anspruch 1, **dadurch gekennzeichnet, dass** die Sedimentation in Gegenwart mindestens eines Sedimentationsbeschleunigers, vorzugsweise einem Polysaccharid und/oder substituiertem Polysaccharid, insbesondere Dextran, Dextransulfat und/oder Hydroxyethylstärke (HES), durchgeführt wird.

3. Verfahren nach Anspruch 1 oder 2, **dadurch gekennzeichnet, dass** eine Sedimentation ohne Zentrifugation durchgeführt wird.

4. Verfahren nach einem der Ansprüche 1 bis 3, **dadurch gekennzeichnet, dass** der Thrombozyteninhalt dadurch freigesetzt wird, dass man die Thrombozyten mit einer wässrigen Flüssigkeit, insbesondere einer wässrigen Lösung, inkubiert, die zu einer Freisetzung der Botenstoffe durch osmotisches Platzen lassen der Thrombozyten führt.

5. Verfahren nach einem der Ansprüche 1 bis 4, **dadurch gekennzeichnet, dass** man den Inhalt der Thrombozyten in einer Freisetzungsflüssigkeit, insbesondere destillierten Wasser, aufnimmt und die erhaltene Lösung des Thrombozyteninhaltes filtriert, insbesondere steril filtriert.

6. Verfahren nach einem der Ansprüche 1 bis 5, **dadurch gekennzeichnet, dass** der Aufschluss der Thrombozyten mit einer Freisetzungsflüssigkeit sowie die Freisetzung der Botenstoffe und insbesondere auch das vorhergehende Waschen der Thrombozyten auf einem Membranfilter, insbesondere einem Sterilfilter, vorgenommen werden.

7. Verwendung eines Membranfilters, insbesondere eines Sterilfilters, mit einer Membran aus Polyethersulfon als Behältnis für den Aufschluss der Thrombozyten und zur Gewinnung einer wundheilungsfördernden Botenstoffmischung aus Thrombozyten enthaltenden Körperflüssigkeiten, insbesondere Blut oder Blutplasma, die frei ist von in wässrigem Lösungsmittel unlöslichen Bestandteilen, insbesondere unlöslichen Blutbestandteilen, wobei sie den wasserlöslichen Inhalt der Thrombozyten mit im Wesentlichen allen löslichen Botenstoffen der Thrombozyten enthält, in Form eines sterilen wässrigen Filtrates.

8. Verwendung nach Anspruch 7, **dadurch gekennzeichnet, dass** sie in Form einer wässrigen Lösung vorliegt.

9. Verwendung nach Anspruch 7, **dadurch gekennzeichnet, dass** sie in lyophilisierter und/oder pulvriger Form vorliegt.

10. Verwendung nach einem der Ansprüche 7 bis 9, **dadurch gekennzeichnet, dass** die Botenstoffmischung, vorzugsweise die wässrige Lösung, in steriler Form und gegebenenfalls steriler Verpackung vorliegt.

11. Verwendung nach einem der Ansprüche 7 bis 10, **dadurch gekennzeichnet, dass** sie frei von Botenstoffe aus Thrombozyten freisetzenden Aktivatoren ist.

12. Verwendung nach einem der Ansprüche 7 bis 11, **dadurch gekennzeichnet, dass** die Botenstoffmischung mit dem wasserlöslichen Zellinhalt in gelösten Zustand eine im Wesentlichen physiologische Lösung ist.

13. Verwendung nach Anspruch 7, **dadurch gekennzeichnet, dass** die Membran eine Porengröße von 0,1 bis 0,5 µm, vorzugsweise von ca. 0,2 µm, besonders bevorzugt von ca. 0,22 µm, hat.

14. Kit zur Gewinnung von wundheilungsfördernden Botenstoffmischungen aus Thrombozyten enthaltenden Körperflüssigkeiten, insbesondere zur Verwendung oder Durchführung des Verfahrens nach einem der Ansprüche 1 bis 6 mit einem
a) Filter, insbesondere Sterilfilter, wobei der Filter, insbesondere Sterilfilter, eine Porengröße von 0,1 µm bis 0,5 µm, vorzugsweise von ca. 0,2 µm, besonders bevorzugt von ca. 0,22 µm, hat,
b) Blutentnahmebesteck mit
c) Blutentnahmesystem, insbesondere einem Blutentnahmeröhrchen, insbesondere einem mit einem Sedimentationsbeschleuniger, vorzugsweise einem Polysaccharid und/oder einem substituierten Polysaccharid, insbesondere Dextran, Dextransulfat und/oder Hydroxyethylstärke (HES), vorgefüllten Blutentnahmeröhrchen oder einem Spritzenzylinder zur Blutaufnahme,
d) Blutgerinnungsinhibitor,

15. Kit nach Anspruch 14 mit
e) einer Leerspritze, insbesondere einer Leerspritze mit einer passenden Kanüle, zur Aufnahme der thrombozytenreichen Fraktion aus dem Überstand,
f) einem Auffanggefäß, insbesondere einem sterile Auffanggefäß, besonders bevorzugt einer sterilen Leerspritze, zur Aufnahme der wundheilungsfördernden Botenstoffe nach Filtrierung, insbesondere nach Sterilfiltrierung.
g) gegebenenfalls einem oder zwei Dreiwegehähnen.

16. Kit nach Anspruch 14 oder 15 mit
h) einer weiteren Spritze, insbesondere einer mit physiologischer Pufferlösung vorgefüllten Spritze.

17. Kit nach einem der Ansprüche 14 bis 16 mit
i) einer weiteren Spritze, insbesondere einer mit Freisetzungslösung vorgefüllten Spritze.

18. Kit nach einem der Ansprüche 14 bis 17, **dadurch gekennzeichnet, dass** der Blutgerinnungsinhibitor in vorzugsweise flüssiger Form in dem Blutentnahmesystem, insbesondere in dem Blutröhrchen oder dem Spritzenzylinder, enthalten ist.

19. Kit nach einem der Ansprüche 14 bis 18, **dadurch gekennzeichnet, dass** der Blutgerinnungsinhibitor in vorzugsweise trockener, insbesondere lyophilisierter Form, in dem Blutentnahmesystem, insbesondere in dem Blutröhrchen oder dem Spritzenzylinder, enthalten ist.

## Claims

1. Method for obtaining a wound healing-promoting mixture of messengers from thrombocyte-containing body fluids, more particularly blood or blood plasma, which is free of aqueous solvent-insoluble constituents, more particularly insoluble blood constituents, wherein the mixture contains the water-soluble content of the thrombocytes with mainly all the soluble messengers of the thrombocytes, wherein the messengers present in collected body fluids, more particularly blood or blood plasma, are obtained, after separation of the body fluids into supernatant and sediment, from the removed supernatant by washing the thrombocytes with buffer solution, releasing the messengers from the thrombocytes and filtering the solution obtained, wherein the thrombocyte content with mainly all the messengers of the thrombocytes is released by disruption of the thrombocytes, **characterized in that** said disruption is performed by lysing the thrombocytes.

2. Method according to Claim 1, **characterized in that** the sedimentation is carried out in the presence of at least one sedimentation accelerator, preferably a polysaccharide and/or substituted polysaccharide, more particularly dextran, dextran sulphate and/or hydroxyethyl starch (HES).

3. Method according to Claim 1 or 2, **characterized in that** sedimentation is carried out without centrifugation.

4. Method according to any of Claims 1 to 3, **characterized in that** the thrombocyte content is released by incubating the thrombocytes with an aqueous liquid, more particularly an aqueous solution, which brings about release of the messengers owing to osmotic bursting of the thrombocytes.

5. Method according to any of Claims 1 to 4, **characterized in that** the content of the thrombocytes is collected in a release liquid, more particularly distilled water, and the thrombocyte-content solution obtained is filtered, more particularly sterile-filtered.

6. Method according to any of Claims 1 to 5, **characterized in that** the disruption of the thrombocytes with a release liquid and the release of the messengers and more particularly also the preceding washing of the thrombocytes are performed on a membrane filter, more particularly a sterile filter.

7. Use of a membrane filter, more particularly a sterile filter, having a membrane composed of polyethersulphone as a vessel for the disruption of thrombocytes and for obtaining a wound healing-promoting mixture of messengers from thrombocyte-containing body fluids, more particularly blood or blood plasma, which is free of aqueous solvent-insoluble constituents, more particularly insoluble blood constituents, wherein the mixture contains the water-soluble content of the thrombocytes with mainly all the soluble messengers of the thrombocytes, in the form of a sterile aqueous filtrate.

8. Use according to Claim 7, **characterized in that** the mixture is present in the form of an aqueous solution.

9. Use according to Claim 7, **characterized in that** the mixture is present in lyophilized and/or powdered form.

10. Use according to any of Claims 7 to 9, **characterized in that** the mixture of messengers, preferably the aqueous solution, is present in sterile form and, where appropriate, in sterile packaging.

11. Use according to any of Claims 7 to 10, **characterized in that** the mixture is free of activators which release messengers from thrombocytes.

12. Use according to any of Claims 7 to 11, **characterized in that** the mixture of messengers with the water-soluble cellular content in a dissolved state is a substantially physiological solution.

13. Use according to Claim 7, **characterized in that** the pore size of the membrane is from 0.1 to 0.5 µm, preferably about 0.2 µm, particularly preferably about 0.22 µm.

14. Kit for obtaining wound healing-promoting mixtures of messengers from thrombocyte-containing body fluids, more particularly for using or carrying out the method according to any of Claims 1 to 6, having a
a) filter, more particularly a sterile filter, wherein the pore size of the filter, more particularly the sterile filter, is from 0.1 µm to 0.5 µm, preferably about 0.2 µm, particularly preferably about 0.22 µm,
b) blood collection apparatus with
c) blood collection system, more particularly a blood collection tube, more particularly a blood collection tube prefilled with a sedimentation accelerator, preferably a polysaccharide and/or a substituted polysaccharide, more particularly dextran, dextran sulphate and/or hydroxyethyl starch (HES), or a syringe cylinder for blood collection,
d) blood coagulation inhibitor.

15. Kit according to Claim 14 having
e) an empty syringe, more particularly an empty syringe having an appropriate needle, for collecting the thrombocyte-rich fraction from the supernatant,
f) a collection vessel, more particularly a sterile collection vessel, particularly preferably a sterile empty syringe, for collecting the wound healing-promoting messengers after filtration, more particularly after sterile filtration,
g) optionally, one or two three-way valves.

16. Kit according to Claim 14 or 15 having
h) a further syringe, more particularly a syringe prefilled with physiological buffer solution.

17. Kit according to any of Claims 14 to 16 having
i) a further syringe, more particularly a syringe prefilled with release solution.

18. Kit according to any of Claims 14 to 17, **characterized in that** the blood coagulation inhibitor is contained in the blood collection system, more particularly in the blood tube or the syringe cylinder, preferably in liquid form.

19. Kit according to any of Claims 14 to 18, **characterized in that** the blood coagulation inhibitor is contained in the blood collection system, more particularly in the blood tube or the syringe cylinder, preferably in dry form, more particularly lyophilized form.

## Revendications

1. Procédé d'obtention d'un mélange de substances messagères, favorisant la cicatrisation, de fluides corporels contenant des thrombocytes, en particulier le sang ou le plasma sanguin, qui est exempt de constituants insolubles dans les solvants aqueux, en particulier de constituants insolubles du sang, le mélange contenant le contenu soluble dans l'eau des thrombocytes avec des substances messagères qui pour l'essentiel sont toutes solubles, les substances solubles présentes dans les fluides corporels prélevés, en particulier le sang ou le plasma sanguin, étant obtenues après séparation des fluides corporels se trouvant dans le surnageant et le sédiment, d'avec le surnageant ainsi séparé, procédé dans lequel on lave les thrombocytes avec une solution tampon, on libère les substances messagères des thrombocytes, et on filtre la solution obtenue, le contenu des thrombocytes, avec pour l'essentiel la totalité des substances messagères des thrombocytes, étant libéré par désagrégation des thrombocytes, **caractérisé en ce que** la désagrégation est mise en oeuvre par lyse des thrombocytes.

2. Procédé selon la revendication 1, **caractérisé en ce que** la sédimentation est mise en oeuvre en présence d'au moins un accélérateur de sédimentation, de préférence un polysaccharide et/ou un polysaccharide substitué, en particulier le dextran, le sulfate de dextran et/ou l'hydroxyéthylamidon (HES).

3. Procédé selon la revendication 1 ou 2, **caractérisé en ce qu'**on met en oeuvre une sédimentation sans centrifugation.

4. Procédé selon l'une des revendications 1 à 3, **caractérisé en ce que** le contenu des thrombocytes est libéré par le fait que l'on incube les thrombocytes avec un liquide aqueux, en particulier une solution aqueuse, qui conduit à une libération des substances messagères par éclatement osmotique des thrombocytes.

5. Procédé selon l'une des revendications 1 à 4, **caractérisé en ce qu'**on reprend le contenu des thrombocytes dans un liquide de libération, en particulier l'eau distillée, et on filtre, en particulier dans des conditions stériles, la solution obtenue du contenu des thrombocytes.

6. Procédé selon l'une des revendications 1 à 5, **caractérisé en ce que** la désagrégation des thrombocytes avec un liquide de libération, ainsi que la libération des substances messagères, et en particulier aussi le lavage préalable des thrombocytes, sont mis en oeuvre sur une membrane filtrante, en particulier un filtre stérile.

7. Utilisation d'une membrane filtrante, en particulier d'un filtre stérile, comportant une membrane en polyéthersulfone en tant que récipient pour la désagrégation des thrombocytes et pour l'obtention d'un mélange de substances messagères favorisant la cicatrisation, constitué de fluides corporels contenant des thrombocytes, en particulier le sang ou le plasma sanguin, qui est exempte de constituants insolubles dans un solvant aqueux, en particulier des constituants insolubles du sang, le mélange contenant le contenu hydrosoluble des thrombocytes, avec pour l'essentiel la totalité des substances messagères solubles des thrombocytes, sous forme d'un filtrat aqueux stérile.

8. Utilisation selon la revendication 7, **caractérisée en ce qu'**elle se présente sous forme d'une solution aqueuse.

9. Utilisation selon la revendication 7, **caractérisée en ce qu'**elle se présente sous forme lyophilisée et/ou pulvérulente.

10. Utilisation selon l'une des revendications 7 à 9, **caractérisée en ce que** le mélange de substances messagères, de préférence la solution aqueuse, se présente sous forme stérile et éventuellement sous forme d'un emballage stérile.

11. Utilisation selon l'une des revendications 7 à 10, **caractérisée en ce qu'**elle est exempte de substances messagères provenant d'activateurs libérant les thrombocytes.

12. Utilisation selon l'une des revendications 7 à 11, **caractérisée en ce que** le mélange de substances messagères, contenant le contenu cellulaire hydrosoluble à l'état dissous, est une solution pour l'essentiel physiologique.

13. Utilisation selon la revendication 7, **caractérisée en ce que** la membrane a une grosseur de pores de 0,1 à 0,5 µm, de préférence d'environ 0,2 µm, d'une manière particulièrement préférée d'environ 0,22 µm.

14. Trousse pour obtenir des mélanges de substances messagères favorisant la cicatrisation, de fluides corporels contenant des thrombocytes, en particulier pour utilisation ou mise en oeuvre du procédé selon l'une des revendications 1 à 6, comportant
a) un filtre, en particulier un filtre stérile, le filtre, en particulier le filtre stérile, ayant une grosseur de pores de 0,1 à 0,5 µm, de préférence d'environ 0,2 µm, d'une manière particulièrement préférée d'environ 0,22 µm,
b) un ensemble de prélèvement de sang, comportant
c) un système de prélèvement du sang, en particulier un tube de prélèvement de sang, en particulier un tube de prélèvement de sang prérempli d'un accélérateur de sédimentation, de préférence un polysaccharide et/ou un polysaccharide substitué, en particulier le dextran, le sulfate de dextran et/ou l'hydroxyéthylamidon (HES), ou un cylindre de seringue pour recueillir le sang,
d) un inhibiteur de coagulation du sang.

15. Trousse selon la revendication 14, comportant
e) une seringue vide, en particulier une seringue vide comportant une canule adaptée, pour recevoir les fractions riches en thrombocytes provenant du surnageant,
f) un récipient récepteur, en particulier un récipient récepteur stérile, d'une manière particulièrement préférée une seringue vide stérile, pour recueillir les substances messagères favorisant la cicatrisation après la filtration, en particulier après la filtration stérile,
g) éventuellement, une ou deux vannes à trois voies.

16. Trousse selon la revendication 14 ou 15, comportant
h) une seringue supplémentaire, en particulier une seringue préremplie d'une solution tampon physiologique.

17. Trousse selon l'une des revendications 14 à 16, comportant
i) une seringue supplémentaire, en particulier une seringue préremplie d'une solution de libération.

18. Trousse selon l'une des revendications 14 à 17, **caractérisée en ce que** l'inhibiteur de coagulation du sang est présent sous forme de préférence liquide dans le système de prélèvement du sang, en particulier dans le tube de prélèvement ou dans le cylindre de seringue.

19. Trousse selon l'une des revendications 14 à 18, **caractérisée en ce que** l'inhibiteur de coagulation du sang se présente sous forme de préférence sèche, en particulier lyophilisée, dans le système de prélèvement du sang, en particulier dans le tube de prélèvement ou dans le cylindre de seringue.
